# EUROPÄISCHE PATENTANMELDUNG

(11) **EP 1 530 974 A1**
(43) Veröffentlichungstag der Anmeldung: **18.05.2005**
(21) Anmeldenummer: 04017137.3
(22) Anmeldetag: 21.07.2004
(51) Int. Cl.: A61L 2/04, A61L 2/26, E02D 3/11, B09C 1/06, A01G 13/02, C09K 17/30

(54) **Verfahren zur Sterilisation von Erdreich**

(30) Priorität: 08.08.2003 DE 10336882
(71) Anmelder: BASF AKTIENGESELLSCHAFT, 67056 Ludwigshafen (DE)
(72) Erfinder: Lehrich, Friedhelm, Dr., 82152 Planegg (DE); Peled, Uri, Zichronjacob 30900 (IL)

(57) **Zusammenfassung**

Die Erfindung betrifft ein Verfahren zur Sterilisation von Erdreich mittels Erwärmung, wobei man das Erdreich mit einer Folie auf der Basis von thermoplastischem Polyurethan abdeckt.

## Beschreibung

Die Erfindung bezieht sich auf Verfahren zur Erwärmung von Erdreich, bevorzugt landwirtschaftlich genutztem Land, besonders bevorzugt Ackerland, insbesondere Gemüseanbauland oder Obstanbauland, insbesondere Erwärmung durch die Sonnenenergie. Außerdem betrifft die Erfindung die Verwendung von Folien auf der Basis von thermoplastischem Polyurethan zur Abdeckung und Erwärmung von Erdreich, bevorzugt landwirtschaftlich genutztem Land, besonders bevorzugt Ackerland, insbesondere Gemüseanbauland oder Obstanbauland.

Im Obst- und Gemüsebau muss vor der Bepflanzung oft eine Entseuchung des Erdreiches vorgenommen werden. Hier gilt es der Pflanze schadende Bakterien wie z.B. Nematoden im Erdreich bis zu 50 cm Tiefe abzutöten.

Es sind zwei Methoden bekannt, welche einzeln oder kombiniert zur Bodensterilisation angewandt werden:
1. Chemische Bodensterilisation:
   Durch Ausbringen von Chemikalien wie z.B. Methylbromid wird das Erdreich sterilisiert. Diese Methode ist sehr wirkungsvoll, allerdings ist Methylbromid toxikologisch nicht unbedenklich. Es kann nicht vollständig abgebaut werden, schädigt das Grundwasser und trägt zum Abbau der Ozonschicht bei. Aus diesen Gründen wird der Einsatz dieses Produktes gemäß dem Montreal Protocol bis spätestens 2010 weltweit verboten sein. Weniger bedenkliche chemische Alternativen zu Methylbromid sind zwar heute am Markt erhältlich, aber nicht wirkungsvoll genug.
2. Physikalische Bodensterilisation:
   Mittels Erwärmung wird das Erdreiches bis zur thermischen Abtötung der Schädlinge erhitzt.

Es ist somit bekannt, Boden physikalisch oder physikalisch/chemisch zu sterilisieren durch Abdeckung mit Folien aus hauptsächlich PE + Additiven aber auch PP + Additiven, PVC + Additiven und ETFE. Diese Folien sollen tagsüber eine hohe Erwärmung des Erdreiches durch die Sonnenenergie zulassen und die so eingebrachte Wärme zu Zeiten von wenig oder keiner zugeführten Sonnenenergie (nachts) im Erdreich halten. So kann sich die Durchschnittstemperatur im Erdreich langsam erhöhen (Prinzip: Treibhauseffekt) bis sie ausreicht um die thermische Abtötung von Schädlingen hervorzurufen. Oben beschriebenen Systeme brauchen heute sehr lange und eine sehr hohe Sonnenintensität um die benötigten Sterilisationstemperaturen zu erreichen und sind somit nicht zuverlässig.

Aufgabe der vorliegenden Erfindung war es, ein einfaches, möglichst schnelles und effizientes sowie günstiges und umweltfreundliches Verfahren zur Sterilisierung von Erdreich, bevorzugt landwirtschaftlich genutztem Land, besonders bevorzugt Ackerland, insbesondere Gemüseanbauland oder Obstanbauland zu entwickeln.

Diese Aufgabe konnte dadurch gelöst werden, dass man das Erdreich mit einer Folie auf der Basis von thermoplastischem Polyurethan abdeckt und durch die Erwärmung der oberen Bodenschicht das Erdreich zumindest partiell sterilisiert.

Durch das erfindungsgemäße Verfahren kann sowohl auf den Einsatz von chemischen Hilfsmitteln verzichtet werden als auch auf eine aufwendige Bodenentfernung und Sterilisierung in extemen Einrichtungen, z.B. Öfen.

Der erfindungsgemäße Einsatz von Folien auf der Basis von thermoplastischem Polyurethan (TPU) bietet den Vorteil, dass im Vergleich zu bekannten Folien eine höhere Temperatur im Erdreich erreicht wird. Mit Folien aus Polyethylen unter Zusatz von IR-Absorbem wird keine befriedigende Temperatur im Erdreich erreicht, damit sind die notwendigen Sterilisationszeiten noch zu lang. Die mit der TPU-Folie erzielbare Temperatur reicht bei guter Sonneneinstrahlung aus, das Erdreich in befriedigender Tiefe zu sterilisieren.

Die thermische Abtötung der Schädlinge ist eine Funktion von Temperatur und Zeit, je niedriger die Temperaturen sind desto länger ist die Sterilisationszeit. Die Sonnenenergie kann auch in warmen Regionen wie Südeuropa den Boden nur kurzzeitig auf Temperaturen im Sterilisationsbereich erwärmen, da im Verlauf eines Tag/Nacht Zyklus das Erdreich zu schnell wieder auskühlt. Für eine wirkungsvolle Sterilisation sollte die über Tag ins Erdreich eingetragene Wärme bevorzugt auch über Nacht im Erdreich gehalten werden. Dieses wird erfindungsgemäß durch die Abdeckung des Bodens mit einer Folie bevorzugt über Nacht erreicht, wodurch das Auskühlen der Erde verzögert wird. Bevorzugt wird das Erdreich bis zu einer Tiefe von 40 cm über eine Zeit von mindestens einer Woche, bevorzugt 1 bis 8 Wochen, besonders bevorzugt 2 bis 3 Wochen auf eine Temperatur von durchschnittlich mindestens 40°C erwärmt. Dafür wird die Folie bevorzugt über eine Dauer von 1 bis 8 Wochen Tagen auf dem Erdreich belassen. Die Folie wird bevorzugt einlagig oder doppellagig direkt auf das Erdreich gelegt und bevorzugt am Rand durch Erde befestigt zur Fixierung und Abdichtung. Die Folie kann auch in einer Lage direkt auf das Erdreich gelegt und die zweite Lage als Tunnel darüber gespannt werden.

Um eine besonders effiziente Erwärmung des Erdreiches zu bewirken, sind Folien bevorzugt, die transparent sind.

Bevorzugt weist die Folie eine Dicke zwischen 0,005 mm und 1 mm, besonders bevorzugt zwischen 0,03 mm und 0,08 mm auf, insbesondere zwischen 0,05 mm und 0,08 mm.

Bevorzugt sind somit Folien, die mindestens 1 Schicht aufweisen, gegebenenfalls eingefärbt sind, bevorzugt aber transparent bis transluzent sind und eine Dicke von 0,005 mm bis 1,000 mm aufweisen.

Als Material zur Herstellung der Folie wird thermoplastisches Polyurethan, im Folgenden auch als TPU bezeichnet, eingesetzt, das allgemein bekannt, vielfältig beschrieben und kommerziell erhältlich ist. Bevorzugt weisen die erfindungsgemäßen TPU eine zumindest teilkristalline Weichphase auf. TPU zeichnen sich unter anderem durch gute Festigkeiten, Abriebe, Weiterreißfestigkeiten und Chemikalienbeständigkeit aus, und können in nahezu beliebiger Härte durch geeignete Rohstoffzusammensetzung hergestellt werden. Zusätzlich bieten TPU den Vorteil einer kostengünstigen Herstellung, beispielsweise nach dem Band- oder dem Reaktionsextruderverfahren, die kontinuierlich oder diskontinuierlich durchgeführt werden können, und die einfache Thermoplastverarbeitung. Die Herstellung von Folien aus dem thermoplastischem Polyurethan beispielsweise mittels Extrusion, z.B. unter Verwendung üblicher Blasköpfe oder Breitschlitzdüsen ist dem Fachmann allgemein bekannt.

Üblicherweise erfolgt die Herstellung durch die Umsetzung von (a) Diisocyanaten, im vorliegenden Fall bevorzugt aliphatischen Diisocyanaten, mit (b) gegenüber Isocyanaten reaktiven Verbindungen mit einem Molekulargewicht von 500 g/mol bis 8000 g/mol gegebenenfalls in Gegenwart von (c) Kettenverlängerungsmitteln mit einem Molekulargewicht von 60 g/mol bis 499 g/mol, (d) Katalysatoren und/oder (e) üblichen Hilfsstoffen.

Zur Einstellung von Härte der TPUs können die Aufbaukomponenten (b) und (c) in relativ breiten molaren Verhältnissen variiert werden. Bewährt haben sich molare Verhältnisse von Komponente (b) zu insgesamt einzusetzenden Kettenverlängerungsmitteln (c) von 1 : 0,5 bis 1 : 8, insbesondere von 1 : 1 bis 1 : 4, wobei die Härte der TPUs mit zunehmendem Gehalt an (c) ansteigt. Die Umsetzung zur Herstellung der TPU kann bei einer Kennzahl von 0,8 bis 1,2 : 1, bevorzugt bei einer Kennzahl von 0,9 bis 1 : 1 erfolgen. Die Kennzahl ist definiert durch das Verhältnis der insgesamt bei der Umsetzung eingesetzten Isocyanatgruppen der Komponente (a) zu den gegenüber Isocyanaten reaktiven Gruppen, d.h. den aktiven Wasserstoffen, der Komponenten (b) und gegebenenfalls (c) und gegebenenfalls monofunktionellen gegenüber Isocyanaten reaktiven Komponenten als Kettenabbruchsmitteln wie z.B. Monoalkoholen. Die Herstellung der thermoplastischen Polyurethane erfolgt üblicherweise im One-shotoder Prepolymerverfahren auf der Bandanlage oder auf dem Reaktionsextruder. Hierbei werden die zur Reaktion kommenden Komponenten (a), (b) und (c) und gegebenenfalls Kettenabbruchsmitteln gemeinsam oder in bestimmter Reihenfolge vereinigt und zur Reaktion gebracht. Beim Reaktionsextruderverfahren werden die Aufbaukomponenten (a) bis (c) sowie gegebenenfalls Kettenabbruchmittel, (d) und/oder (e) einzeln oder als Gemisch in den Extruder eingeführt, z.B. bei Temperaturen von 100 bis 250°C, vorzugsweise 140 bis 220°C zur Reaktion gebracht, das erhaltene TPU wird extrudiert, abgekühlt und granuliert. Die Herstellung der TPU-Folie kann nach allgemein bekannten Verfahren erfolgen.

Die bei der Herstellung der TPUs üblicherweise verwendeten Komponenten (a), (b), (c) sowie gegebenenfalls (e) und/oder (f) sollen im Folgenden beispielhaft beschrieben werden:
a) Als Isocyanate, üblicherweise Diisocyanate, können aliphatische, cycloaliphatische, araliphatische und/oder aromatische Diisocyanate eingesetzt werden. Im einzelnen seien beispielhaft die folgenden aromatische Isocyanate genannt: 2,4-Toluylen-diisocyanat, Gemische aus 2,4- und 2,6-Toluylen-diisocyanat, 4,4'-, 2,4'- und/oder 2,2'-Diphenylmethan-diisocyanat, Gemische aus 2,4'- und 4,4'―Diphenylmethan―diisocyanat, urethanmodifizierte flüssige 4,4'- und/oder 2,4-Diphenylmethan-diisocyanate, 4,4'―Diisocyanato―diphenylethan― (1,2) und 1 ,5-Naphthylen-diisocyanat. Als aliphatische Diisocyanate (a) werden übliche aliphatische und/oder cycloaliphatische Diisocyanate eingesetzt, beispielsweise Tri-, Tetra-, Penta-, Hexa-, Hepta- und/oder Oktamethylendiisocyanat, 2-Methylpentamethylen―diisocyanat―1 ,5, 2―Ethyl―butylen―diisocyanat―1,4, 1-Isocyanato-3,3,5-trimethyl-5-isocyanatomethyl-cyclohexan (Isophoron―diisocyanat, IPDI), 1,4- und/oder 1,3-Bis(isocyanatomethyl)cyclohexan (HXDI), 1,4-Cyclohexandiisocyanat, 1-Methyl-2,4- und/oder ―2,6―cyclohexan―diisocyanat, 4,4'-, 2,4'- und/oder 2,2'-Dicyclohexylmethan-diisocyanat. Bevorzugt wird Hexamethylen-1,6-diisocyanat (Hexamethylendiisocyanat, HDI) und/oder 4,4'-, 2,4'- und/oder 2,2'―Diphenylmethan―diisocyanat (MDI) als Isocyanat (a) eingesetzt.
b) Als gegenüber Isocyanaten reaktive Verbindungen (b) können allgemein bekannte Polyhydroxylverbindungen mit Molekulargewichten von 500 g/mol bis 8000 g/mol, bevorzugt 800 g/mol bis 6000 g/mol, insbesondere 2000 g/mol bis 4000 g/mol, und bevorzugt einer mittleren Funktionalität von 1,8 bis 2,6, bevorzugt 1,9 bis 2,2, insbesondere 2 eingesetzt werden, beispielsweise allgemein bekannte Polyesterole, Polyetherole und/oder Polycarbonatdiole. Bevorzugt werden als (b) ε-Caprolacton und/oder Polyesterdiol auf der Basis von Adipinsäure und Butan-1,4-diol und/oder Hexan-1,6-diol als Diolkomponente eingesetzt, wobei das Verhältnis von Butan-1,4-diol und Hexan-1,6-diol in Abhängigkeit des gewünschten Schmelzpunktes der Weichphase und der gewünschten Kristallinität der Weichphase gewählt werden kann. Besonders bevorzugt wird Polytetrahydrofuran, bevorzugt mit einem Molekulargewicht zwischen 800 und 2000 g/mol als (b) eingesetzt.
c) Als Kettenverlängerungsmittel (c) können allgemein bekannte Verbindungen eingesetzt werden, beispielsweise Diamine und/oder Alkandiole mit 2 bis 10 C-Atomen im Alkylenrest, insbesondere Ethylenglykol und/oder Butandiol-1,4, und/oder Hexandiol und/oder Di- und/oder Tri-oxyalkylenglykole mit 3 bis 8 Kohlenstoffatomen im Oxyalkylenrest, bevorzugt entsprechende Oligo-Polyoxypropylenglykole, wobei auch Mischungen der Kettenverlängerer eingesetzt werden können. Als Kettenverlängerer können auch 1,4-Bis-(hydroxymethyl)-benzol (1,4-BHMB), 1,4-Bis-(hydroxyethyl)-benzol (1,4-BHEB) oder 1,4-Bis-(2-hydroxyethoxy)-benzol (1,4-HQEE) zum Einsatz kommen. Bevorzugt werden als Kettenverlängerungsmittel Ethylenglykol, Butandiol und/oder Hexandiol eingesetzt.
d) Geeignete Katalysatoren, welche insbesondere die Reaktion zwischen den NCO-Gruppen der Diisocyanate (a) und den Hydroxylgruppen der Aufbaukomponenten (b) und (c) beschleunigen, sind die nach dem Stand der Technik bekannten und üblichen tertiären Amine, wie z.B. Triethylamin, Dimethylcyclohexylamin, N―Methylmorpholin, N,N'-Dimethylpiperazin, 2-(Dimethylaminoethoxy)-ethanol, Diazabicyclo-(2,2,2)-octan und ähnliche sowie insbesondere organische Metallverbindungen wie Titansäureester, Eisenverbindungen wie z.B. Eisen-(III)-acetylacetonat, Zinnverbindungen, z.B. Zinndiacetat, Zinndioctoat, Zinndilaurat oder die Zinndialkylsalze aliphatischer Carbonsäuren wie Dibutylzinndiacetat, Dibutylzinndilaurat oder ähnliche. Die Katalysatoren werden üblicherweise in Mengen von 0,0001 bis 0,1 Gew.-Teiten pro 100 Gew.-Teile Polyhydroxylverbindung (b) eingesetzt.
e) Neben Katalysatoren können den Aufbaukomponenten (a) bis (d) auch übliche Hilfsstoffe (e) hinzugefügt werden. Genannt seien beispielsweise oberflächenaktive Substanzen, Glasfasem, Flammschutzmittel, Keimbildungsmittel, Gleit- und Entformungshilfen, Farbstoffe und Pigmente, Inhibitoren, Stabilisatoren gegen Hydrolyse, Licht, Hitze, Oxidation oder Verfärbung, Schutzmittel gegen mikrobiellen Abbau, anorganische und/oder organische Füllstoffe, Verstärkungsmittel und Weichmacher. Genannt seien auch Additive, die speziell in dieser genannten Erfindung zu einer Haftverbesserung zwischen TPU und Holz, Spanplatte bzw. Hoizersatzstoffen führen. Solche Haftverbesserer können z.B. Isocyanat enthaltende Additive sein.
   Genannte Hilfsstoffe bzw. Additive können dem TPU direkt bei der Synthese oder erst bei der thermoplastischen Verarbeitung in Substanz oder eingearbeitet in einem Träger, z.B. TPU, als sogenannte Masterbatches zugesetzt werden.

Neben den genannten Rohstoffen (a)-(e) können auch Kettenabbruchsmittel mit einem Molekulargewicht von 46 bis 499 eingesetzt werden. Solche Kettenabbruchsmittel sind Verbindungen, die lediglich eine gegenüber Isocyanaten reaktive funktionelle Gruppe aufweisen, wie z.B. Monoalkohole. Durch solche Kettenabbruchsmittel kann das Fließverhalten gezielt eingestellt werden.

Nähere Angaben über die oben genannten Hilfsmittel- und Zusatzstoffe sind der Fachliteratur zu entnehmen. Alle in dieser Schrift genannten Molekulargewichte weisen die Einheit [g/mol] auf und stellen das Zahlenmittel des Molekulargewichtes dar.

Erfindungsgemäß besonders bevorzugt ist eine Folie auf der Basis von thermoplastischem Polyurethan, die die folgenden Eigenschaften aufweist: gute Lichtdurchlässigkeit für UV vis Strahlung sowie eine hohe Absorption /Reflektion von IR Strahlung bevorzugt im Wellenzahlbereich 500 bis 1500 cm⁻¹.

Bevorzugt ist die Folie UV stabilisiert. Bevorzugt ist die Folie mit einem üblichen Antifog Additiv ausgerüstet. Bevorzugt weist die Folie eine hohe Reiß- und Weiterreißfestigkeit auf.
Gerade eine solche Folie hat sich als besonders geeignet herausgestellt in der Landwirtschaftlichen Nutzung, da thermoplastische Polyurethane bereits eine natürlich gute Lichtdurchlässigkeit für UV vis Strahlung sowie eine hohe Absorption /Reflektion von IR Strahlung bevorzugt im Wellenzahlbereich 500 bis 1500 cm⁻¹ aufweisen.

Das erfindungsgemäße Verfahren beruht somit darauf, dass die Folie tagsüber eine möglichst hohe Erwärmung des Erdreiches durch die Sonnenenergie zulässt und nachts eine Auskühlung des Erdreiches verhindert um die Durchschnittstemperatur im Erdreich zu Erhöhen (Prinzip des Treibhauseffektes).

## Patentansprüche

1. Verfahren zur Sterilisation von Erdreich mittels Erwärmung, **dadurch gekennzeichnet, dass** man das Erdreich mit einer Folie auf der Basis von thermoplastischem Polyurethan abdeckt.

2. Verfahren gemäß Anspruch 1, **dadurch gekennzeichnet, dass** das Erdreich in einer Tiefe von 40 cm über eine Zeit von mindestens einer Woche auf eine Temperatur von durchschnittlich mindestens 40 °C erwärmt wird.

3. Verfahren gemäß Anspruch 1, **dadurch gekennzeichnet, dass** die Folie transparent ist.

4. Verfahren gemäß Anspruch 1, **dadurch gekennzeichnet, dass** die Folie eine Dicke zwischen 0,005 mm und 1 mm aufweist.

5. Verfahren gemäß Anspruch 1, **dadurch gekennzeichnet, dass** die Folie UV-stabilisiert ist und ein Antifog Additiv enthält.

6. Verwendung von Folien auf der Basis von thermoplastischem Polyurethan zur Abdeckung und Erwärmung von Erdreich.
